(19) **Europäisches Patentamt**
**European Patent Office**
**Office européen des brevets**

(11) **EP 1 662 981 B1**

(12) **EUROPÄISCHE PATENTSCHRIFT**

(45) Veröffentlichungstag und Bekanntmachung des Hinweises auf die Patenterteilung:
**16.12.2009 Patentblatt 2009/51**

(21) Anmeldenummer: **04764869.6**

(22) Anmeldetag: **06.09.2004**

(51) Int Cl.:
*A61B 3/107* *(2006.01)*    *A61B 3/103* *(2006.01)*

(86) Internationale Anmeldenummer:
**PCT/EP2004/009921**

(87) Internationale Veröffentlichungsnummer:
**WO 2005/027741 (31.03.2005 Gazette 2005/13)**

(54) **MESSUNG DER OBERFLÄCHENTOPOGRAPHIE UND WELLENABERRATION EINES LINSENSYSTEMS**

DEVICE AND METHOD FOR MEASURING THE SURFACE TOMOGRAPHY AND WAVE ABERRATION OF A LENS SYSTEM, PARTICULARLY AN EYE

DISPOSITIF ET PROCEDE POUR MESURER LA TOPOGRAPHIE SUPERFICIELLE ET L'ABERRATION D'ONDE D'UN SYSTEME A LENTILLE, NOTAMMENT D'UN OEIL

(84) Benannte Vertragsstaaten:
**AT BE BG CH CY CZ DE DK EE ES FI FR GB GR HU IE IT LI LU MC NL PL PT RO SE SI SK TR**

(30) Priorität: **12.09.2003 DE 10342175**

(43) Veröffentlichungstag der Anmeldung:
**07.06.2006 Patentblatt 2006/23**

(73) Patentinhaber: **Optocraft GmbH**
**91098 Erlangen (DE)**

(72) Erfinder:
• **BEYERLEIN, Mathias**
**91052 Erlangen (DE)**

• **PFUND, Johannes**
**90425 Nürnberg (DE)**

(74) Vertreter: **Dörr, Matthias**
**Tergau & Pohl**
**Patentanwälte**
**Mögeldorfer Hauptstrasse 51**
**90482 Nürnberg (DE)**

(56) Entgegenhaltungen:
**DE-A- 19 713 138    US-A1- 2002 163 623**

**Beschreibung**

Die Erfindung bezieht sich auf eine Vorrichtung zur Messung der Oberflächentopographie (nachfolgend kurz "Topographie") und Wellenaberration eines Linsensystems, mit einem ersten Messsystem, das eine Lichtquelle zur Ausstrahlung eines ersten Lichtbündels einer ersten Wellenlänge sowie einen Detektor zur Aufnahme des an dem Linsensystem reflektierten ersten Lichtbündels umfasst, sowie mit einem zweiten Messsystem, das eine Lichtquelle zur Ausstrahlung eines zweiten Lichtbündels einer zweiten Wellenlänge sowie einen Detektor zur Aufnahme des durch das Linsensystem transmittierten zweiten Lichtbündels umfasst. Eine derartige Vorrichtung kann grundsätzlich zur Vermessung eines jeglichen optischen Linsensystems verwendet werden. Insbesondere kommt ein solche Vorrichtung aber in der optischen Medizintechnik zum Einsatz. Bei dem zu untersuchenden Linsensystem handelt es sich hierbei um ein (insbesondere menschliches) Auge. Die Erfindung bezieht sich weiterhin auf ein zugehöriges Verfahren.

Für chirurgische Eingriffe am menschlichen Auge, wie beispielsweise die Korrektur der Fehlsichtigkeit des Auges, wird in jüngerer Zeit vermehrt die Ablation von Cornea-Gewebe mittels Excimer-Laser eingesetzt. Dazu wird eine lappenartige Schicht der Cornea (d.h. der transparenten Augenhornhaut) aufgeschnitten und zur Seite geklappt. Daraufhin wird am frei liegenden Cornea-Gewebe ein geeigneter Betrag an Gewebe ablatiert (d.h. abgetragen) und danach der Cornea-Lappen wieder replatziert. Auf diese Weise wird die Cornea gezielt derart deformiert, dass eine Fehlsichtigkeit des Auges, z.B. eine Kurz- oder Weitsichtigkeit oder ein Astigmatismus, kompensiert ist. Um die abzutragende Menge an Cornea-Gewebe mit hinreichender Genauigkeit berechnen zu können, ist ein detaillierte Kenntnis sowohl der Wellenaberration, d.h. der optischen Fehlabbildung des Auges als auch der Topographie der Cornea erforderlich.

Eine ähnliche Informationsdichte ist auch bei anderen Korrekturverfahren am menschlichen Auge, z.B. der Transplantation der Cornea, dem Austausch der Augenlinse gegen eine Kunstlinse oder der Anpassung einer Kontaktlinse erforderlich.

Bisher werden die Topographie der Cornea und die Wellenaberration des Auges üblicherweise getrennt gemessen. Dadurch ergeben sich häufig Diskrepanzen zwischen den beiden in zeitlicher Abfolge durchgeführten Messungen aufgrund der Instabilität des Auges als biologisches Objekt einerseits und andererseits aufgrund der zahlreichen Justage-Freiheitsgrade des Auges relativ zur Messeinrichtung. Bei der Anwendung in der optischen Medizin kann eine solche Messdiskrepanz insbesondere den Erfolg eines chirurgischen Eingriffs am Auge oder eines sonstigen medizinischen Korrekturverfahrens beeinträchtigen.

Zur Vermeidung solcher Diskrepanzen ist daher eine simultane Messung der Topographie und der Wellenaberration wünschenswert. Dies ist an sich bei einem aus der US 2002/0163623 A1 bekannten Messverfahren ermöglicht. Nach dem bekannten Verfahren und der zugehörigen Vorrichtung sind zwei Messsysteme vorgesehen, die Lichtsignale unterschiedlicher Wellenlänge aussenden und nach Reflexion am Auge bzw. an der Cornea wieder auffangen. Bei der bekannten Vorrichtung ist die Strahlführung der für die beiden Messungen benötigten Lichtbündel nur vergleichsweise aufwändig zu realisieren. Dies begrenzt umgekehrt die Präzision der Messungen.

Ein ähnliches Messverfahren ist weiterhin aus der US 2001/0016695 A1 bekannt.

Der Erfindung liegt die Aufgabe zugrunde, eine Vorrichtung und ein Verfahren zur Messung der Topographie und Wellenaberration eines Linsensystems zu verbessern.

Bezüglich der Vorrichtung wird die Aufgabe erfindungsgemäß gelöst durch die Merkmale des Anspruchs 1. Bezüglich des zugehörigen Verfahrens wird die Aufgabe erfindungsgemäß gelöst durch die Merkmale des Anspruchs 17. Danach ist in einem gemeinsamen Strahlengangbereich eines ersten und eines zweiten Messsystems ein diffraktives (d.h. Lichtbeugung verursachendes) optisches Element angeordnet, das den jeweiligen Wellenfrontverlauf eines ersten Lichtbündels und eines zweiten Lichtbündels wellenlängenselektiv anpaßt.

Als Wellenfrontverlauf wird die Funktion des dreidimensionalen Raums bezeichnet, die an jedem Ort innerhalb des Strahlengangs des jeweils betrachteten Lichtbündels die räumliche Ausrichtung einer auf die örtliche Lichtausbreitungsrichtung senkrechten Fläche beschreibt. Das diffraktive optische Element (DOE) wirkt insofern wellenlängenselektiv, als der Wellenfrontverlauf der beiden Lichtbündel aufgrund deren unterschiedlicher Wellenlänge beim Durchgang durch das DOE auf unterschiedliche Weise beeinflußt wird.

Durch die Nutzung des DOE, und der damit ermöglichten wellenlängenselektiven Strahlführung lässt sich der Wellenfrontverlauf der beiden Lichtbündel flexibel, quasi unabhängig und hochpräzise den Anforderungen der jeweiligen Messung anpassen.

In einer bevorzugten Ausführung der Erfindung ist das DOE derart ausgebildet, dass die nullte Beugungsordnung des ersten Lichtbündels unterdrückt, d.h. in ihrer Intensität vollständig ausgelöscht oder zumindest stark erniedrigt wird. Das Licht des ersten Lichtbündels wird vielmehr überwiegend oder vollständig in die erste Beugungsordnung transmittiert. Gleichzeitig wird durch geeignete Ausführung des DOE erreicht, dass es auf das Licht des zweiten Lichtbündels im Wesentlichen keine beugende Wirkung ausübt. Das zweite Lichtbündel wird somit im Wesentlichen ungeschwächt in die nullte Beugungsordnung transmittiert.

Durch eine geeignete Ausführung des DOE wird zweckmäßigerweise der Wellenfrontverlauf des durch das DOE in Richtung des Linsensystems transmittierten ersten Lichtbündels an die Topographie des Linsenytems vorangepasst. Unter Voranpassung wird insbesondere verstanden, dass der Wellenfrontverlauf des ersten Lichtbündels durch das DOE in einer Weise deformiert wird, dass die Krümmung der Wellenfronten am Ort des Linsensystems etwa dessen Oberflächenkrümmung entsprechen. Dies hat den Vorteil, dass das Licht des ersten Lichtbündels überall im Wesentlichen senkrecht auf die Oberfläche des Linsensystems auftrifft und annähernd in sich zurückgeworfen wird. Auf diese Weise können schon geringe Abweichungen der Topographie des Linsensystems von der Voranpassung präzise erfasst werden. Außerdem wird durch die Voranpassung des Wellenfrontverlaufes der Lichtverlust durch Streulicht reduziert. In Anwendung der Erfindung auf das menschliche Auge als zu prüfendes Linsensystem ist das DOE bevorzugt derart gestaltet, dass der Wellenfrontverlauf des ersten Lichtbündels an ein medizinisches Standardmodell des menschlichen Auges, insbesondere das Gullstrandsche Normal-Auge angepasst wird. Im Hinblick auf das zweite Lichtbündel ist das DOE dagegen bevorzugt derart ausgebildet, dass dessen Wellenfrontverlauf im Wesentlichen nicht modifiziert wird.

Besonders geeignet als DOE ist ein oberflächen-korrugiertes Phasenelement. Hierunter wird eine Platte aus Glas oder einem transparenten Kunststoff verstanden, in deren Oberfläche ein reliefartiges Beugungsgitter eingebracht ist. Mittels computergestützter Herstellungsverfahren und geeigneter Ätztechniken, kann ein solches Phasenelement heutzutage vergleichsweise preisgünstig mit äußerst hoher Präzision hergestellt werden. Hierbei kann die Beugungswirkung des Phasenelements hochflexibel an den Bedarf angepasst werden. Mit einem oberflächen-korrigierten Phasenelement ist insbesondere ein Oberflächengitter mit äußerst kleiner Gitterperiode in der Größenordnung weniger hundert Nanometer und damit ein vergleichsweise großer Ablenkwinkel des gebeugten Lichts erreichbar. Prinzipiell ist es jedoch denkbar, dass DOE auf andere Weise, z.B. durch ein Volumenhologramm oder ein reflektives diffraktives Element zu realisieren. Insbesondere ist es auch denkbar, das DOE als frei ansteuerbares, flexibles optisches Element auszuführen. Dies ist beispielsweise durch phasenschiebende Flüssigkristallanzeigen (LCD) möglich.

Um die Brechkraft und die Ausrichtung eines zu untersuchenden Auges während der Dauer der Messung zu fixieren, und somit die Messgenauigkeit zu erhöhen, ist bevorzugt vorgesehen, durch ein drittes Lichtbündel ein Fixationstarget in das Auge einzublenden. Unter einem Fixationstarget wird ein Bild verstanden, das einem zu untersuchenden Probanden während der Messung zur Betrachtung angeboten wird. Indem der Proband das Fixationstarget anvisiert, hält er automatisch sowohl die Orientierung des Auges als auch die vom Auge eingestellte Brechkraft in guter Näherung konstant. Das zur Einblendung des Fixationstargets herangezogene dritte Lichtbündel hat eine dritte Wellenlänge, für die bevorzugt das DOE ebenfalls wirkungslos ist. Die dritte Wellenlänge ist bevorzugt sowohl von der ersten Wellenlänge als auch von der zweiten Wellenlänge verschieden. Auf diese Weise ist gewährleistet, dass das dritte Lichtbündel weder die Topographiemessung, noch die Wellenaberrationsmessung beeinflußt. In einer vereinfachten Ausführung der erfindungsgemäßen Vorrichtung ist jedoch alternativ vorgesehen, dass die dritte Wellenlänge der zweiten Wellenlänge entspricht.

In einer besonders rationellen Variante der Vorrichtung teilen die beiden Messsysteme einen gemeinsamen Detektor.

Zur Erzielung großer Beugungswinkel, sowie um zu vermeiden, dass ein zu untersuchender Proband durch die Lichtbündel während der Messung geblendet wird, liegen die Wellenlängen des ersten und/oder zweiten Lichtbündels bevorzugt im langwelligen und für das menschliche Auge nicht sichtbaren Nahinfrarotbereich des elektromagnetischen Spektrums. Die erste Wellenlänge ist insbesondere etwa im Bereich zwischen 1000 nm und 1600 nm gewählt. Die zweite Wellenlänge entspricht bevorzugt in grober Näherung der Hälfte der ersten Wellenlänge. Die zweite Wellenlänge liegt dementsprechend im Grenzbereich zwischen dem sichtbarem roten Spektralbereich und dem Nahinfrarotbereich.

Zur Vermeidung von Messfehlern, die durch eine Fehljustierung des Linsensystems bezüglich des DOE hervorgerufen werden, umfasst die Vorrichtung zweckmäßigerweise eine Justageanordnung, mit welcher die Position des Linsensystems bestimmt und eingestellt werden kann. Eine besonders vorteilhafte Realisierung einer solchen Justageanordnung umfasst eine Lichtquelle sowie einen positionssensitiven Detektor. Zur Justierung des Linsensystems wird ein Justagelichtstrahl von der Lichtquelle unter einem Winkel auf das Linsensystem geworfen, und der daran reflektierte Justagelichtstrahl auf dem positionssensitiven Detektor aufgefangen. Zur Justage des Linsensystems wird nun die Position des Linsensystems bezüglich des DOE variiert, bis der reflektierte Justagelichtstrahl auf einem vorbestimmten Punkt der Detektorfläche auftrifft, der die korrekte Justierung des Linsensystems bezüglich des DOE anzeigt.

Für eine besonders präzise Anpassung des Wellenfrontverlaufs der Lichtbündel ist es vorteilhaft, wenn das DOE dem Linsensystem unmittelbar vorgeschaltet ist, so dass das Licht der Lichtbündel ausgehend von dem DOE direkt auf das Linsensystem fällt. Umgekehrt fällt das vom Linsensystem zurückgeworfene Licht der Lichtbündel wieder direkt auf das DOE, bevor es den Detektoren der beiden Messsysteme zugeleitet wird.

Durch einen, den gemeinsamen Strahlengangbereich der beiden Messsysteme begrenzenden, wellenlängenselektiven Strahlteiler wird erreicht, dass die beiden Lichtbündel nach Reflexion am Auge effektiv dem jeweiligen Detektor zugeführt werden. Hierdurch wird insbesondere eine unerwünschte Interaktion der beiden Messsysteme vermieden, die zu einer Verfälschung des Messergebnisses führen könnte. In einer zweckmäßigen Ausführung der Erfindung ist ein solcher Strahlteiler wiederum als diffraktives optisches Element ausgebildet, das den Strahlengang der beiden Lichtbündel wellenlängenselektiv voneinander trennt.

Als Detektor des ersten und/oder des zweiten Messsystems ist insbesondere ein so genannter Wellenfrontdetektor geeignet. Hierunter versteht man einen Detektor, der die örtliche Ausrichtung einer Wellenfront erfasst. Im Rahmen der erfindungsgemäßen Vorrichtung ist insbesondere der Einsatz eines Shack-Hartmann-Sensors oder eines Interferometers, z.B. von Shearing-Typ, als Detektor des ersten und/oder zweiten Messsystems vorgesehen. Weiterhin vorteilhaft im Rahmen der erfindungsgemäßen Vorrichtung als Detektor einsetzbar sind ein Pyramidal-Sensor oder ein Talbot-Interferometer.

Im Zuge des mit der erfindungsgemäßen Vorrichtung durchgeführten Verfahrens erfolgt die Messung der Topographie und die Messung der Wellenaberration bevorzugt simultan. Hierdurch wird zum einen eine besonders kurze Messzeit erreicht. Dies wiederum ist insbesondere bei der Anwendung des Verfahrens am menschlichen Auge von Vorteil, zumal ein zu untersuchender Proband während der Messung möglichst unbeweglich verharren muss, was notwendigerweise mit einer gewissen Unannehmlichkeit verbunden ist. Zum anderen wird durch die simultane Messung der Topographie und der Wellenaberration Diskrepanzen zwischen dem jeweiligen Ergebnis dieser beiden Messmethoden vermieden, die durch die Instabilität des menschlichen Auges bei zeitlich beabstandeten Messungen auftreten würden.

Eine zeitlich sequentielle, d.h. eine (insbesondere in sehr kurzem Abstand) zeitlich versetzte Messung ist dennoch als im Hinblick auf eine vereinfachte Verfahrensführung vorteilhafte Alternative vorgesehen. Dies ist besonders bei Verwendung eines für beide Messsysteme gemeinsamen Detektors zur besseren Trennung der Messsignale der beiden Messsysteme sinnvoll. Zur Vermeidung von Messdiskrepanzen werden die Messungen bevorzugt in einem zeitlichen Abstand durchgeführt, der die Reaktionszeit des Auges unterschreitet, so dass die Messungen im Hinblick auf das Auge quasi-simultan erfolgt.

In einer zweckmäßigen alternativen Ausführung des erfindungsgemäßen Verfahrens wird die Messung der Topographie und/oder die Messung der Wellenaberration mit Hilfe eines so genannten Scanning-Spot-Verfahrens durchgeführt. Bei dieser an sich herkömmlichen Messtechnik wird das Linsensystem nicht mit einem flächigen Lichtbündel beleuchtet, sondern mit einem feinen, im Querschnitt annähernd punktförmigen Lichtstrahl abgetastet. Entsprechend wird bei einem Scanning-Spot-Verfahren nicht der Wellenfrontverlauf des vom Linsensystem reflektierten Lichts, sondern die Ablenkung des reflektierten dünnen Lichtstrahls von einer für ein "ideales Linsensystem" charakteristischen Sollposition gemessen. Hinsichtlich des Informationsgehalts über die Topographie oder die Wellenaberration des untersuchten Linsensystems sind ein gewöhnliches Wellenfrontverfahren und ein Scanning-Spot-Verfahren äquivalent.

Nachfolgend werden Ausführungsbeispiele der Erfindung anhand einer Zeichnung näher erläutert. Darin zeigen:

Fig. 1 in schematischer Darstellung eine Vorrichtung zur Messung der Topographie und Wellenaberration eines Linsensystems, insbesondere des menschlichen Auges, mit einem in einem gemeinsamen Strahlengangbereich eines ersten Messsystems und eines zweiten Messsystems angeordneten diffraktiven optischen Element (DOE),

Fig. 2 in einem schematisch vergrößerten und ausschnittsweise gezeigten Querschnitt das diffraktive optische Element gemäß Fig. 1,

Fig. 3 in schematischer Darstellung das diffraktive optische Element und das Auge im Strahlengang eines ersten Lichtbündels zur Messung der Topographie des Auges,

Fig. 4 in Darstellung gemäß Fig. 3 das diffraktive optische Element und das Auge im Strahlengang eines zweiten Lichtbündels zur Messung der Wellenaberration des Auges,

Fig. 5 in Darstellung gemäß Fig. 1 eine alternative Ausführung der Vorrichtung und

Fig. 6 bis 8 in Darstellung gemäß Fig. 1 weitere Ausführungsformen der Vorrichtung.

Einander entsprechende Teile und Größen sind in den Figuren stets mit dem gleichen Bezugszeichen versehen.

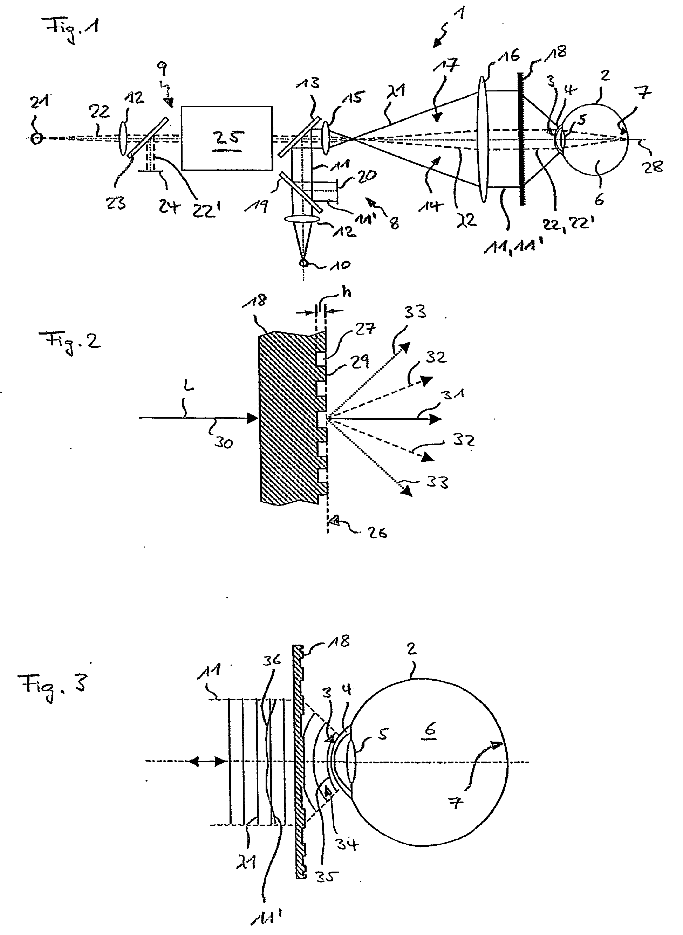

Fig. 1 zeigt in einer schematischen Skizze eine Vorrichtung 1 zur Messung der Topographie und Wellenaberration eines Linsensystems. Unter den Begriff Linsensystem fällt hierbei jedes künstliche oder natürliche, mit einer oder mehreren Linsen ausgestattete optische System. Bei dem in Fig. 1 schematisch dargestellten Linsensystem handelt es sich insbesondere um ein menschliches Auge 2.

Als (Oberflächen-)Topographie wird die dreidimensionale Form der Linsenoberfläche bezeichnet. Im Falle des Auges 2 ist diese Linsenoberfläche die Oberfläche 3 der Cornea 4, d.h. der transparenten Augenhornhaut. Das Linsensystem des Auges 2 umfasst weiterhin in bekannter Weise die Augenlinse 5 und den Glaskörper 6. In dem der Augenlinse 5 entgegengesetzten Augenhintergrund ist in bekannter Weise die Retina 7 (oder Netzhaut) angeordnet.

Unter dem Begriff Wellenaberration wird allgemein die Abweichung der optischen Abbildungseigenschaften des zu prüfenden realen Linsensystems von den Abbildungseigenschaften eines entsprechenden idealen Linsensystems bezeichnet. Im Falle des Auges 2 umfasst die Wellenaberration Abbildungsfehler erster Ordnung wie Kurzsichtigkeit, Weitsichtigkeit oder Stabsichtigkeit (Astigmatismus) sowie Abbildungsfehler höherer Ordnung.

Zur Messung der Topographie der Cornea 4 ist die Vorrichtung 1 mit einem ersten Messsystem 8 versehen.

Zur Messung der Wellenaberration ist ein zweites Messsystem 9 vorgesehen.

Das erste Messsystem 8 umfasst eine Lichtquelle 10, insbesondere einen Laser. Die Lichtquelle 10 erzeugt ein erstes Lichtbündel 11 einer ersten Wellenlänge $\lambda 1$. Das Lichtbündel 11 wird entlang des Strahlengangs des ersten Messsystems 8 zunächst in einer Kollimatorlinse 12 parallel gerichtet und mittels eines wellenlängenselektiven Strahlteilers 13 in einen gemeinsamen Strahlengangbereich 14 der Messsysteme 8 und 9 eingestrahlt. Innerhalb des Strahlengangbereichs 14 wird das erste Lichtbündel 11 durch ein aus zwei Linsen 15 und 16 bestehendes Kepler-Teleskop 17 aufgeweitet und durchtritt ein dem Auge 2 unmittelbar vorgeschaltetes diffraktives optisches Element, nachfolgend kurz als DOE 18 bezeichnet. Durch das nachfolgend in seiner Funktionsweise näher beschriebene DOE 18 wird das erste Lichtbündel 11 in Richtung des Auges 2 kollimiert. Ein Anteil des auf das Auge 2 einfallenden Lichtbündels 11 (nachfolgend vereinfachend als reflektiertes Lichtbündel 11' bezeichnet), wird an der Oberfläche 3 der Cornea 4 reflektiert und entgegen der Einfallsrichtung durch das DOE 18, das Kepler-Teleskop 17 und den Strahlteiler 13 zurückgeworfen. Durch einen außerhalb des gemeinsamen Strahlengangbereichs 14 angeordneten weiteren Strahlteiler 19 wird das reflektierte Lichtbündel 11' aus dem einfallenden Lichtbündel 11 ausgekoppelt und auf einen Wellenfrontdetektor 20 geleitet. Das Kepler-Teleskop 17 ist dabei derart ausgebildet, das die Cornea 4 auf dem Wellenfrontdetektor 20 scharf abgebildet wird. Der Wellenfrontdetektor 20 ist wahlweise als Shack-Hartmann-Sensor ausgeführt, wie er beispielsweise in der US 2003/0038921 A1 beschrieben ist. Alternativ kann der Wellenfrontdetektor 20 auch als Interferometer, insbesondere Shearing-Interferometer, ausgeführt sein.

Das zweite, zur Messung der Wellenaberration vorgesehene Messsystem 9 umfasst eine weitere Lichtquelle 21. Die Lichtquelle 21, die wiederum vorzugsweise durch einen Laser realisiert ist, emittiert ein zweites Lichtbündel 22 einer zweiten Wellenlänge $\lambda 2$ in Form eines vergleichsweise feinen Lichtbündels. Das zweite Lichtbündel 22 wird wiederum in einer Kollimatorlinse 12 parallelgerichtet und durch den wellenlängenselektiven Strahlteiler 13 in den gemeinsamen Strahlengangbereich 14 eingestrahlt. Infolge seiner Wellenlängenselektivität ist der Strahlteiler 13 für die Wellenlänge $\lambda 2$ transparent, mithin wirkungslos. Ein Strahlteiler 13 mit derartiger Wellenlängenselektivität ist nach herkömmlicher Technik beispielsweise durch einen dielektrischen Spiegel herstellbar.

Im weiteren Verlauf seines Strahlengangs fällt das zweite Lichtbündel 22 durch das Kepler-Teleskop 17 und das DOE 18 hindurch auf das Auge 2. Das DOE 18 ist hierbei in nachfolgend näher beschriebener Weise derart gestaltet, dass es für Licht der Wellenlänge $\lambda 2$ keine oder nur vernachlässigbare beugende Wirkung entfaltet. Das Lichtbündel 22 durchsetzt infolgedessen das DOE 18 quasi unmodifiziert und fällt als weiterhin feines Lichtbündel durch die Cornea 4 und die Augenlinse 5 auf die Retina 7. Das Lichtbündel 22 wird an der Retina 7 diffus zurückgestreut. Dieses Streulicht, nachfolgend als zurückgestreutes Lichtbündel 22' bezeichnet, fällt durch die Augenlinse 5, die Cornea 4, das DOE 18, das Kepler-Teleskop 17 und den für die Wellenlänge $\lambda 2$ transparenten Strahlteiler 13 entgegen seiner Einfallsrichtung zurück. Durch einen außerhalb des gemeinsamen Strahlengangbereichs 14 im Strahlengang des Lichtbündels 22,22' positionierten weiteren Strahlteiler 23 wird das zurückgestreute Lichtbündel 22' ausgekoppelt und auf einen Wellenfrontdetektor 24 des zweiten Messystems 9 geworfen. Der Wellenfrontdetektor 24 ist wiederum wahlweise als Shack-Hartmann-Sensor oder als Interferometer ausgeführt. Den Strahlteilern 13 und 23 ist eine Vorkompensationseinheit 25 zwischengeschaltet. Diese Vorkompensationseinheit 25 enthält ein (nicht näher dargestelltes) herkömmliches optisches Zoom-System oder eine Linsenanordnung, mit welcher die Anteile Defokus und Astigmatismus, d.h. die Kurz- oder Weitsichtigkeit und die Stabsichtigkeit, kompensierbar ist. Die Vorkompensationseinheit 25 dient umkehrt auch dazu, das einfallende Lichtbündel 22 scharf auf der Retina 7 abzubilden.

Anders als bei dem Auge 2 kann bei einem künstlichen Linsensystem die Wellenaberrationsmessung im Allgemeinen vereinfacht durchgeführt werden, indem das Linsensystem zwischen Lichtquelle und Detektor angeordnet wird, so dass das Linsensystem nur einfach von dem zweiten Lichtbündel durchleuchtet wird.

Das in Fig. 1 dargestellte DOE 18 ist ein so genanntes oberfächen-korrugiertes Phasenelement, dessen Aufbau und Funktionsweise in Fig. 2 schematisch skizziert ist. Das abgebildete DOE 18 ist ein Plättchen aus Glas oder einem transparenten Kunststoff, in dessen dem Auge 2 zugewandte Oberfläche 26 ein reliefartiges Beugungsgitter eingebracht ist. Das Beugungsgitter des DOE 18 umfasst eine Anzahl von Vertiefungen 27, die näherungsweise kreisringförmig ausgebildet und näherungsweise konzentrisch um die optische Achse 28 (Fig. 1) der Vorrichtung 1 angeordnet sind und durch zwischenliegende Stege 29 voneinander getrennt sind. Zur Erzielung asphärischer Beugungsmuster können die Vertiefungen 27 und die zwischenliegenden Stege 29 in vorbestimmter Weise von der sphärischen Form und konzentrischen Anordnung abweichen.

Wie aus Fig. 2 zu erkennen ist, besitzen bevorzugt alle Vertiefungen 27 die gleiche Strukturtiefe h, so dass die Oberfläche 26 in zwei diskrete Niveaus gegliedert ist. Man bezeichnet ein solchermaßen aufgebautes DOE auch als binäres Hologramm.

Die Vertiefungen 27 und Stege 29 der reliefartigen Oberfläche 26 bilden hinsichtlich ihrer optischen Wirkung alternierende Phasen, innerhalb derer sich eine einfallende ebene Lichtwelle L infolge der Brechungsindexdifferenz zwischen dem Material des DOE 18 und der umgebenden Luft unterschiedlich entwickelt. Hierdurch entsteht beim Durchtritt der Lichtwelle L durch die Oberfläche 26 ein Phasenunterschied zwischen Teilwellen im Bereich der Vertiefungen 27 und Teilwellen im Bereich der Stege 29, die in an sich bekannter Weise zu Interferenz- und Beugungseffekten führen.

Diese Effekte haben zur Folge, dass die eingestrahlte Lichtwelle L beim Austritt aus dem DOE 18 nur in diskrete Richtungen bezüglich der Einfallrichtung 30 abgestrahlt wird. Die in diese diskreten Richtungen abgestrahlten Teilstrahlen werden als Beugungsordnungen 31,32,33 bezeichnet. Im Allgemeinen wird ein Teil der eingestrahlten Strahlungsintensität ungebeugt, d.h. in Einfallsrichtung 30, abgestrahlt. Diese Teilstrahlung wird als nullte Beugungsordnung 31 bezeichnet. Mit steigendem Winkel bezüglich dieser nullten Beugungsordnung 31 werden die weiteren Beugungsmaxima als erste Beugungsordnung 32, zweite Beugungsordnung 33 usw. bezeichnet. Höhere Beugungsordnungen sind in der schematischen Darstellung gemäß Fig. 2 aus Gründen der Vereinfachung nicht dargestellt.

Die Winkel, unter denen die einzelnen Beugungsordnungen 32,33, usw. bezüglich der nullten Beugungsordnung 31 erscheinen, sind von der Gitterkonstante des Beugungsgitters, d.h. dem Abstand zweier benachbarter Vertiefungen 27 abhängig. Die Beugungswinkel sind hierbei um so größer, je kleiner die Gitterkonstante ist.

Durch geeignete Gestaltung des DOE 18 im Hinblick auf die Wellenlänge der eingestrahlten Lichtwelle L kann erreicht werden, dass das eingestrahlte Licht bevorzugt in eine bestimmte Beugungsordnung 31,32 oder 33 abgestrahlt wird. Dies wird erfindungsgemäß durch Abstimmung des Beugungsgitters des DOE 18 mit den Wellenlängen $\lambda 1$ und $\lambda 2$ zur wellenlängenselektiven Anpassung des Wellenfrontverlaufs der Lichtbündel 11 und 22 ausgenutzt.

Das DOE 18 ist dabei derart auf die Wellenlänge $\lambda 1$ abgestimmt, dass Teilwellen des Lichtbündels 11 im Bereich einer Vertiefung 27 einerseits und im Bereich eines Stegs 29 andererseits gerade um ein ungeradzahliges Vielfaches der halben Wellenlänge versetzt die Oberfläche 26 durchstoßen, und somit in Einfallrichtung 30, d.h. entlang der optischen Achse 38, negativ interferieren. Diese Bedingung ist dann erfüllt, wenn die Strukturtiefe h der Gleichung

$$h = i \cdot \frac{\lambda_1}{2 \cdot (n(\lambda_1) - 1)} \qquad \text{Glg. 1}$$

genügt, wobei $n(\lambda_1)$ der Brechungsindex des Materials des DOE 18 für die Wellenlänge $\lambda_1$ und $i = 1,3,5,...$ eine ungerade, natürliche Zahl ist.

Das DOE 18 ist weiterhin so gestaltet, dass die von einer Vertiefung 27 eingenommene Fläche im Wesentlichen der Fläche eines angrenzenden Stegs 29 entspricht. Hierdurch wird erreicht, dass die nullte Beugungsordnung 31 des ersten Lichtbündels 11 quasi vollständig durch destruktive Interferenz unterdrückt wird.

Wie aus Fig. 3 zu erkennen ist, bildet sich bei Beleuchtung des DOE 18 mit dem Lichtbündel 11 durch den vorstehend beschriebenen Beugungseffekt im Bereich des Auges 2 ein gekrümmter Wellenfrontverlauf 34 aus. Durch geeignete Variation des Gitterabstands des DOE 18 wird dieser Wellenfrontverlauf 34 an das Auge 2 vorangepasst, so dass die Krümmung der Wellenfronten 35 im Bereich der Cornea 4 der durchschnittlichen Oberflächenkrümmung der menschlichen Cornea entspricht. Optional ist das DOE 18 derart ausgebildet, dass der gekrümmte Wellenfrontverlauf 34 einer sphärischen Welle entspricht. Eine sphärische Welle enthält insbesondere einen Fokus, anhand dessen eine vergleichsweise einfache Kalibrierung des gesamten Messsystems 8 möglich ist. Um eine asphärische Abweichung der Corneaoberfläche mit vergleichsweise einfachen Mitteln voranzupassen, ist das DOE 18 alternativ derart ausgebildet, dass der gekrümmte Wellenfrontverlauf 34 einer sphärischen Welle mit einem an die Cornea 4 angepassten konischen Anteil entspricht. Wiederum alternativ ist vorgesehen, das DOE 18 derart auszubilden, dass die Form der Wellenfronten 35 am Ort der Cornea 4 der aus dem Standard-Augenmodell von Gullstrand abzuleitenden Durchschnittsform der Cornea 4 entspricht.

Würde die Topographie der Cornea 4 exakt der Voranpassung, insbesondere der durch das Gullstrandsche Normal-Auge vorgegebenen Krümmung, entsprechen, so würde das Lichtbündel 11 überall exakt senkrecht auf die Oberfläche 3 der Cornea 4 auftreffen und exakt in sich auf das DOE 18 zurückgeworfen. Das DOE 18 würde in diesem Idealfall in Umkehrung des vorbeschriebenen Beugungseffekts den gekrümmten Wellenfrontverlauf des reflektierten Lichtbündels 11' in eine ebene Welle zurückwandeln, die dem einfallenden Lichtbündel 11 exakt entspräche.

Die Topographie der Cornea 4 des realen Auges 2 ist jedoch individuell verschieden und weicht insbesondere mehr oder weniger stark von dem Gullstrandschen Normal-Auge ab. Infolgedessen wird der Wellenfrontverlauf 34 des Lichtbündels 11 bei der Reflexion an der Oberfläche 3 verzerrt. Das reflektierte Lichtbündel 11' wird dementsprechend beim Durchgang durch das DOE 18 in eine gegenüber der einfallenden ebenen Wellenfront verkrümmte Wellenfront 36 umgesetzt. Die Krümmung der Wellenfront 36 wird im Wellenfrontdetektor 20 erfasst. Hieraus kann mit an sich bekannten rechnerischen Methoden die Topographie der Cornea 4 berechnet werden.

Das DOE 18 ist andererseits derart auf die Wellenlänge $\lambda 2$ abgestimmt, dass das zweite Lichtbündel 22 bevorzugt in die nullte Beugungsordnung 31 transmittiert wird. Dies ist immer dann der Fall, wenn zwischen Vertiefungen 27 und angrenzenden Stegen 29 eine Phasendifferenz von einem ganzzahligen Vielfachen der vollen Wellenlänge $\lambda 2$ erreicht wird. Bedingung hierfür ist, dass die Strukturtiefe der Gleichung

$$h = j \cdot \frac{\lambda_2}{(n(\lambda_2)-1)} \qquad\qquad \text{Glg. 2}$$

genügt, wobei $n(\lambda_2)$ der Brechungsindex des Materials des DOE 18 für die Wellenlänge $\lambda_2$ und $j = 1,2,3,....$ eine natürliche Zahl ist.

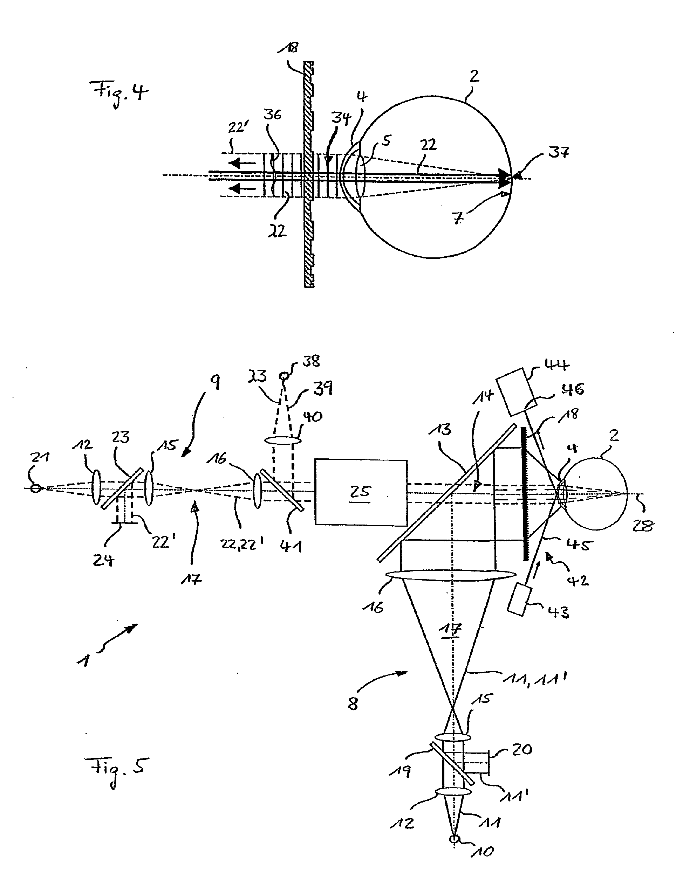

Ist diese Bedingung erfüllt, so wird der Wellenfrontverlauf 34 des Lichtbündels 22 beim Durchgang durch das DOE 18, wie aus Fig. 4 erkennbar, quasi nicht modifiziert. Das DOE 18 ist somit für das Lichtbündel 22 im Wesentlichen wirkungslos.

Die Messung der Wellenaberration des Auges wird gemäß Fig. 4 derart vorgenommen, dass mit dem als feiner Strahl ausgebildeten Lichtbündel 22 ein annähernd punktförmiger Fleck 37 auf der Retina 7 beleuchtet wird. Das von diesem Fleck diffus zurückgestreute Lichtbündel 22' fällt durch die Augenlinse 5 und die Cornea 4 auf das DOE 18 zurück und wird von dort in Richtung des Wellenfrontdetektors 24 transmittiert.

Infolge seines geringen Strahlquerschnitts ist das einfallende Lichtbündel 22 nur in vergleichsweise geringem Maße von der Wellenaberration des Auges 2 beeinflußt. Das rückgestreute Lichtbündel 22' durchsetzt auf dem Rückweg aber die volle Querschnittsfläche der Augenpupille und sammelt demzufolge die gesamte Information über die Wellenaberation des Auges 2 auf.

Während bei idealer Abbildungscharakteristik des Auges 2, d.h. bei verschwindender Wellenaberration, erwartet würde, dass das von dem beleuchteten Fleck 37 als quasi punktförmiger Lichtquelle ausgehende Lichtbündel 22' durch das (entspannte) Auge 2 in eine ebene Welle mit parallelen Wellenfronten abgebildet werden sollte, sind die Wellenfronten 36 des Lichtbündels 22' in aller Regel durch die nicht-verschwindende Wellenaberration des realen Auges 2 verkrümmt. Diese Verkrümmung wird in der Vorkompensationseinheit 25 in erster Ordnung (durch Korrektur einer Kurz-, Weit- oder Stabsichtigkeit) vorkompensiert. Die nach der Vorkompensation verbleibende Verkrümmung höherer Ordnung der Wellenfront 36 wird durch den Wellenfrontdetektor 24 erfasst. Hieraus wird mit an sich bekannten Methoden die Wellenaberration des Auges 2 berechnet.

Die Wellenlängen $\lambda1$ und $\lambda2$ sind zur Erzielung großer Beugungswinkel bevorzugt im vergleichsweise langwelligen Infrarotbereich gewählt. Das nicht-sichtbare Infrarotlicht hat zudem den Vorteil, dass die Messung sowohl der Topographie als auch der Wellenaberration unbemerkt von dem Probanden durchgeführt werden kann. Dadurch wird insbesondere vermieden, dass der Proband durch die Lichtbündel 11 oder 22 geblendet wird oder in die Messung beeinträchtigender Weise auf die Lichteinstrahlung reagiert. Vorteilhafte Wellenlängenkombinationen sind insbesondere $\lambda1 = 1550\,\text{nm}$ und $\lambda2 = 785\,\text{nm}$. Unter dem Gesichtspunkt der besseren Verfügbarkeit sensitiver und vergleichsweise preisgünstiger Lichtsensoren ist andererseits auch die Wahl kleinerer Wellenlängen für das erste Lichtbündel 11 und/ oder das zweite Lichtbündel 22 vorteilhaft; insbesondere $\lambda1 = 1064\,\text{nm}$ und/oder $\lambda2 = 532\,\text{nm}$. Die Topographiemessung und die Wellenaberrationsmessung erfolgen bevorzugt simultan, können aber auch einzeln oder zeitversetzt hintereinander durchgeführt werden.

In Fig. 5 ist eine alternative Ausführungsform der Vorrichtung 1 gezeigt. Diese Ausführungsform unterscheidet sich von der Ausführung gemäß Fig. 1 dadurch, dass der Strahlteiler 13 hier unmittelbar an das DOE 18 anschließt. Die Lichtbündel 11 und 22 werden somit erst unmittelbar, bevor sie auf das DOE 18 fallen, in dem gemeinsamen Strahlengangbereich 14 zusammengeführt bzw. die Lichtbündel 11',22' auf dem Rückweg direkt nach Durchlaufen des DOE 18 getrennt. Außerhalb des gemeinsamen Strahlengangs 14 ist im Strahlengang beider Lichtbündel 11,11' und 22,22' je ein Kepler-Teleskop 17 zur Aufweitung bzw. Abbildung der Lichtbündel 11,11';22,22' angeordnet.

In der Ausführung gemäß Fig. 5 umfasst die Vorrichtung 1 eine dritte Lichtquelle 38, durch welche ein drittes Lichtbündel 39 einer dritten Wellenlänge $\lambda3$ dem Auge 2 eingeblendet wird. Das dritte Lichtbündel 39 wird wiederum durch eine Kollimatorlinse 40 parallelgerichtet und mittels eines wellenlängenselektiven Strahlteilers 41 auf die optische Achse 28 und damit auf das Auge 2 zu ausgerichtet. Das dritte Lichtbündel 39 dient dazu, dem Auge 2 ein so genanntes Fixationstarget anzubieten. Hierunter versteht man ein Bild, das der Proband während der Messung anvisiert. Durch das Anvisieren des Fixationstargets wird zum einen die Sehachse des Auges 2 entlang der optischen Achse 28 ausgerichtet. Zum andere wird die Brechkraft der Augenlinse 5 in einem Bereich fixiert, in welchem der Proband das Fixationstarget scharf erkennen kann. Insbesondere wird dem Probanden häufig durch das Fixationstarget ein Bild im Unendlichen vorgetäuscht, so dass die Augenlinse 5 während der Messung im entspannten Zustand gehalten wird. Das dritte Lichtbündel 39 durchläuft ebenfalls die Vorkompensationseinheit 25, um insbesondere eine eventuelle Kurzsichtigkeit des Auges 2 auszugleichen, und so dem Probanden überhaupt die Möglichkeit zu geben, das Fixationstarget scharf anzuvisieren. Die Wellenlänge $\lambda3$ des dritten Lichtbündels 39 muss notwendigerweise im sichtbaren Spektralbereich liegen und ist bevorzugt derart ausgewählt, dass das DOE 18 keine beugende Wirkung auf das Lichtbündel 39 ausübt. Die dritte Wellenlänge $\lambda3$ kann damit zur Vereinfachung des Messaufbaus insbesondere auch gleich der zweiten Wellenlänge λ2 gewählt sein. In diesem Fall wird das dritte Lichtbündel 39 während der Wellenaberrationsmessung kurzzeitig ausgeblendet. Alternativ kann die dritte Wellenlänge λ3 jedoch auch derart gewählt sein, dass das DOE 18 die nullte Beugungsordnung 31 des dritten Lichtbündels 39 unterdrückt.

Sogenannte Dejustageaberrationen können auch durch eine falsche Positionierung des Auges 2 in Bezug auf dessen laterale und axiale Stellung bezüglich des DOE 18 hervorgerufen werden. Das Auge 2 muss deshalb vor Beginn der Messung exakt bezüglich der Vorrichtung 1 justiert werden. Analog zu herkömmlichen Messvorrichtungen der optischen Medizin wird der Proband für die Messung durch (nicht näher dargestellte) Anlageflächen für Kinn und Stirn bezüglich der Vorrichtung 1 fixiert. Da die Abmessungen des Kopfes jedoch von Proband zu Proband variieren, ist eine Feinjustage der Vorrichtung 1 bezüglich des fixierten Kopfes des Probanden zur richtigen Positionierung des Auges 2 erforderlich. Zur Erleichterung dieses Justagevorgangs dient eine in Fig. 5 schematisch dargestellte Justageanordnung 42, die eine Lichtquelle 43 und einen positionssensitiven Detektor 44 umfasst. Durch die Lichtquelle 43, die insbesondere ein Laser ist, wird ein feiner Justagelichtstrahl 45 schräg auf das Auge 2 geworfen und an der Cornea 4 in Richtung des positionssensitiven Detektors 44 reflektiert. Bei korrekter Justierung des Auges 2 trifft der Justagelichtstrahl 45 in einem vorbestimmten Punkt des Detektors 44 auf. Ist das Auge 2 dagegen dejustiert, so trifft der reflektierte Justagelichtstrahl 45 in einem davon verschiedenen Punkt oder - bei grober Dejustage des Auges 2 - überhaupt nicht auf dem Detektor 44 auf. Vor Beginn der Messung wird deshalb die Lage der Vorrichtung 1 bezüglich des Auges 2 so lange verstellt, bis der Auftreffpunkt des reflektierten Justagelichtstrahls 45 mit dem vorbestimmten Punkt 46 auf dem Detektor 44 übereinstimmt. Bei dem Detektor 44 handelt es sich im einfachsten Fall um einen Sichtschirm, auf welchem der Auftreffpunkt des Justagelichtstrahls per Auge beobachtet werden kann. Der Detektor 44 kann aber auch ein elektronischer Detektor, insbesondere ein CCD-Sensor sein.

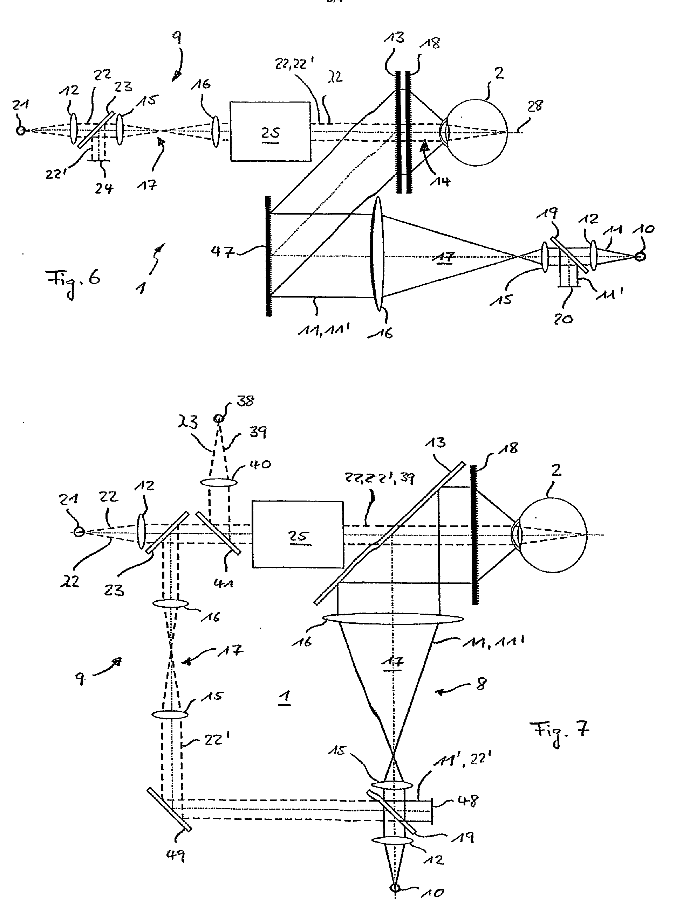

Eine weitere, in Fig. 6 dargestellte Ausführungsform der Vorrichtung 1 unterscheidet sich von den vorstehend beschriebenen Ausführungen dadurch, dass der wellenlängenselektive Strahlteiler 13 ebenfalls als diffraktives optisches Element ausgeführt ist. In gleicher Weise wie das DOE 18 beugt der Strahlteiler 13 hierbei selektiv die Wellenlänge λ1, während Licht der Wellenlänge λ2, insbesondere das Lichtbündel 22, den Strahlteiler 13 ungebeugt transmittiert. Der Strahlteiler 13 weist eine Oberflächen- oder Volumenstruktur in Form eines regelmäßigen Lineargitters auf, wodurch der Strahlengang des ersten Lichtbündels 11,11' um einen definierten Winkel umgelenkt wird. Der Strahlteiler 13 und das DOE 18 können hierbei auch in einem einzelnen optischen Element vereint sein. Durch ein weiteres DOE 47 wird der Strahlengang des Lichtbündels 11,11' wieder in eine, zur optischen Achse 28 parallele Richtung umgelenkt. Das DOE 47 ist hierbei im Gegensatz zu dem Strahlteiler 13 und dem DOE 18 als reflektives Element, insbesondere als Sägezahn-Prisma, ausgebildet.

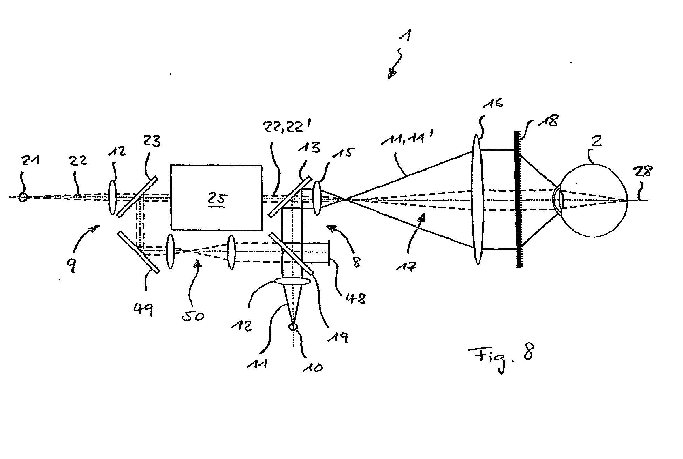

Bei den in den Fig. 7 und 8 dargestellten weiteren Varianten der Vorrichtung 1 ist ein gemeinsamer Detektor 48 für beide Messsysteme 8 und 9 vorgesehen. Dieser besonders rationelle Aufbau wird dadurch ermöglicht, dass das zurückgestreute zweite Lichtbündel 22' nach seiner Auskopplung aus der optischen Achse 28 durch einen Spiegel 49 in Richtung des Strahlteilers 19 des ersten Messsystems 8 umgelenkt wird. Der Strahlteiler 19 ist in dieser Ausführung, ähnlich wie der Strahlteiler 13, frequenzselektiv und insofern transparent für die Wellenlänge λ2 ausgeführt, um eine quasi ungeschwächte Transmission des Lichtbündels 22' auf den Detektor 48 sicherzustellen.

Das gemäß Fig. 7 im Strahlengang des zweiten Lichtbündels 22' angeordnete Kepler-Teleskop 17 erfüllt die zusätzliche Aufgabe, das Lichtbündel 22' aufzuweiten, um die Detektionsfläche des Detektors 48 auszunützen und somit die Auflösung des Detektors 48 auszuschöpfen. Dem gleichen Zweck dient ein gemäß Fig. 8 zusätzlich in dem Strahlengang des zweiten Lichtbündels 22' angeordnetes weiteres Kepler-Teleskop 50.

Um die von dem gemeinsamen Detektor 48 erfassten Signalanteile der beiden Messsysteme 8 und 9 voneinander trennen zu können, werden die Messungen der Topographie und der Wellenaberration bevorzugt zeitlich sequentiell durchgeführt. Die beiden Messungen erfolgen dabei derart kurz nacheinander, dass das Auge 2 während der gesamten Messdauer quasi statisch bleibt. Auf der Zeitskala einer typischen Reaktionszeit des Auges 2 erfolgen die Messungen der Topographie und der Wellenaberration somit quasi-simultan. Eine simultane Messung der Topographie und Wellenaberration ist jedoch auch mit dem gemeinsamen Detektor 48 möglich, sofern dieser einen farbempfindlichen Sensor, z.B. einen RGB-Sensor, enthält. Die Signalanteile der beiden Messsysteme 8 und 9 können dann infolge der unterschiedlichen Wellenlänge λ1 bzw. λ2 der beiden Lichtbündel 11' und 22' nach der Detektion getrennt werden.

Bezugszeichenliste

| 1 | Vorrichtung |
|---|---|
| 2 | Auge |
| 3 | Oberfläche |
| 4 | Cornea |
| 5 | Augenlinse |

| 6 | Glaskörper |
|---|---|
| 7 | Retina |
| 8 | (erstes) Messsystem |
| 9 | (zweites) Messsystem |
| 10 | Lichtquelle |
| 11,11' | (erstes) Lichtbündel |
| 12 | Kollimatorlinse |
| 13 | Strahlteiler |
| 14 | (gemeinsamer) Strahlengangbereich |
| 15 | Linse |
| 16 | Linse |
| 17 | Kepler-Teleskop |
| 18 | diffraktives optisches Element (DOE) |
| 19 | Strahlteiler |
| 20 | Wellenfrontdetektor |
| 21 | Lichtquelle |
| 22,22' | (zweites) Lichtbündel |
| 23 | Strahlteiler |
| 24 | Wellenfrontdetektor |
| 25 | Vorkompensationseinheit |
| 26 | Oberfläche |
| 27 | Vertiefung |
| 28 | optische Achse |
| 29 | Steg |
| 30 | Einfallrichtung |
| 31 | Nullte Beugungsordnung |
| 32 | Erste Beugungsordnung |
| 33 | Zweite Beugungsordnung |
| 34 | Wellenfrontverlauf |
| 35 | Wellenfront |
| 36 | Wellenfront |
| 37 | Fleck |
| 38 | Lichtquelle |
| 39 | Lichtbündel |
| 40 | Kollimatorlinse |
| 41 | Strahlteiler |
| 42 | Justageanordnung |
| 43 | Lichtquelle |
| 44 | (positionssensitiver) Detektor |
| 45 | Justagelichtstrahl |
| 46 | Punkt |
| 47 | Diffraktives optisches Element (DOE) |
| 48 | Detektor |
| 49 | Spiegel |
| 50 | Kepler-Teleskop |

| $\lambda 1$ | Wellenlänge |
|---|---|
| $\lambda 1'$ | Wellenlänge |
| $\lambda 2$ | Wellenlänge |
| $\lambda 3$ | Wellenlänge |
| L | Lichtwelle |
| h | Strukturtiefe |

**Patentansprüche**

1. Vorrichtung (1) zur Messung der Topographie und Wellenaberration eines Linsensystems (2),

- mit einem ersten Messsystem (8), das eine Lichtquelle (10) zur Ausstrahlung eines ersten Lichtbündels (11) einer ersten Wellenlänge ($\lambda 1$) sowie einen Detektor (20,48) zur Aufnahme des an dem Linsensystem (2) reflektierten ersten Lichtbündels (11') umfasst, sowie
- mit einem zweiten Messsystem (9), das eine Lichtquelle (21) zur Ausstrahlung eines zweiten Lichtbündels (22) einer zweiten Wellenlänge ($\lambda 2$) sowie einen Detektor (24,48) zur Aufnahme des durch das Linsensystem (2) transmittierten zweiten Lichtbündels (22') umfasst,

**dadurch gekennzeichnet,**
**dass** in einem gemeinsamen Strahlengangbereich (14) des ersten Messsystems (8) und des zweiten Messsystems (9) ein diffraktives optisches Element (18) angeordnet ist, welches den jeweiligen Wellenfrontverlauf (34) des ersten Lichtbündels (11,11') und des zweiten Lichtbündels (22,22') wellenlängenselektiv anpaßt.

2. Vorrichtung (1) nach Anspruch 1,
   **dadurch gekennzeichnet,**
   **dass** das diffraktive optische Element (18) dahingehend ausgebildet ist, dass die nullte Beugungsordnung (31) des ersten Lichtbündels (11,11') unterdrückt ist, während das zweite Lichtbündel (22,22') im Wesentlichen ungestört in die nullte Beugungsordnung (31) transmittiert wird.

3. Vorrichtung (1) nach Anspruch 1 oder 2,
   **dadurch gekennzeichnet,**
   **dass** das diffraktive optische Element (18) dahingehend ausgebildet ist, dass der Wellenfrontverlauf (34) des ersten Lichtbündels (11,11') an die Topographie des Linsensystems (2) vorangepasst wird.

4. Vorrichtung (1) nach einem der Ansprüche 1 bis 3,
   **dadurch gekennzeichnet,**
   **dass** der Wellenfrontverlauf (34) des zweiten Lichtbündels (22,22') durch das diffraktive optische Element (18) im Wesentlichen nicht modifiziert wird.

5. Vorrichtung (1) nach einem der Ansprüche 1 bis 4,
   **dadurch gekennzeichnet,**
   **dass** das diffraktive optische Element (18) als oberflächen-korrugiertes Phasenelement ausgebildet ist.

6. Vorrichtung (1) nach einem der Ansprüche 1 bis 5,
   **gekennzeichnet durch**
   eine dritte Lichtquelle (38) zur Ausstrahlung eines dritten Lichtbündels (39) einer dritten Wellenlänge ($\lambda 3$), das zur Einblendung eines Fixationstargets in das Linsensystem (2) ausgebildet ist.

7. Vorrichtung (1) nach Anspruch 6,
   **dadurch gekennzeichnet,**
   **dass** die dritte Wellenlänge ($\lambda 3$) der zweiten Wellenlänge ($\lambda 2$) entspricht.

8. Vorrichtung (1) nach einem der Ansprüche 1 bis 7,
   **dadurch gekennzeichnet,**
   **dass** für das erste Messsystem (8) und das zweite Messsystem (9) ein gemeinsamer Detektor (48) vorgesehen ist.

9. Vorrichtung (1) nach einem der Ansprüche 1 bis 8,
   **dadurch gekennzeichnet,**
   **dass** zumindestens die erste Wellenlänge ($\lambda 1$) im infraroten Spektralbereich liegt.

10. Vorrichtung (1) nach einem der Ansprüche 1 bis 9,
    **gekennzeichnet durch**
    eine Justageanordnung (42) zur Einstellung der Position des Linsensystems (2) bezüglich des diffraktiven optischen Elements (18).

11. Vorrichtung (1) nach Anspruch 10,
    **dadurch gekennzeichnet,**
    **dass** die Justageanordnung (42) eine Lichtquelle (43), mittels der ein Justagelichtstrahl (45) unter einem Winkel auf das Linsensystem (2) geworfen werden kann, sowie einen positionssensitiven Detektor (44) zur Lagebestimmung des am Linsensystem (2) reflektierten Justagelichtstrahls (45) umfasst.

12. Vorrichtung (1) nach einem der Ansprüche 1 bis 11,
**dadurch gekennzeichnet,**
**dass** das diffraktive optische Element (18) dem Linsensystem (2) unmittelbar vorgeschaltet ist.

13. Vorrichtung (1) nach einem der Ansprüche 1 bis 12,
**dadurch gekennzeichnet,**
**dass** der gemeinsame Strahlengangbereich (14) durch einen wellenlängenselektiven Strahlteiler (13) begrenzt ist.

14. Vorrichtung (1) nach Anspruch 13,
**dadurch gekennzeichnet,**
**dass** der Strahlteiler (13) als diffraktives optisches Element ausgebildet ist.

15. Vorrichtung (1) nach einem der Ansprüche 1 bis 14,
**dadurch gekennzeichnet,**
**dass** der Detektor (20,48) des ersten Messsystems (8) und/oder der Detektor (24,48) des zweiten Messsystems (9) ein Shack-Hartmann-Sensor ist.

16. Vorrichtung (1) nach einem der Ansprüche 1 bis 15,
**dadurch gekennzeichnet,**
**dass** der Detektor (20,48) des ersten Messsystems (8) und/oder der Detektor (24,48) des zweiten Messsystems (9) ein Interferometer ist.

17. Verfahren zur Messung der Topographie und Wellenaberration eines Linsensystems (2) bei welchem zur Messung der Topographie ein erstes Lichtbündel (11) einer ersten Wellenlänge ($\lambda$1) auf das Linsensystem (2) geworfen, und das an dem Linsensystem (2) reflektierte erste Lichtbündel (11') detektiert wird, bei welchem zur Messung der Wellenaberration ein zweites Lichtbündel (22) einer zweiten Wellenlänge ($\lambda$2) auf das Linsensystem (2) geworfen, und das durch das Linsensystem (2) transmittierte zweite Lichtbündel (22') detektiert wird,
**dadurch gekennzeichnet,**
**dass** durch ein in einem gemeinsamen Strahlengangbereich (14) des ersten Lichtbündels (11,11') und des zweiten Lichtbündels (22,22') positioniertes diffraktives optisches Element (18) der jeweilige Wellenfrontverlauf (34) des ersten Lichtbündels (11,11') und des zweiten Lichtbündels (22,22') wellenlängenselektiv angepasst wird.

18. Verfahren nach Anspruch 17,
**dadurch gekennzeichnet,**
**dass** die erste Wellenlänge ($\lambda$1) und die zweite Wellenlänge ($\lambda$2) im Hinblick auf das das diffraktive optische Element (18) derart gewählt sind, dass die nullte Beugungsordnung (31) des ersten Lichtbündels (11,11') durch das diffraktive optische Element (18) unterdrückt wird, während das zweite Lichtbündel (22,22') im Wesentlichen ungeschwächt in die nullte Beugungsordnung (31) transmittiert wird.

19. Verfahren nach Anspruch 17 oder 18,
**dadurch gekennzeichnet,**
**dass** durch das diffraktive optische Element (18) der Wellenfrontverlauf (34) des ersten Lichtbündels (11) an die Topographie des Linsensystems (2) vorangepasst wird.

20. Verfahren nach einem der Ansprüche 17 bis 19,
**dadurch gekennzeichnet,**
**dass** die Messung der Topographie und die Messung der Wellenaberration simultan erfolgen.

21. Verfahren nach einem der Ansprüche 17 bis 20,
**dadurch gekennzeichnet,**
**dass** die Messung der Topographie und die Messung der Wellenaberration zeitlich sequentiell erfolgen.

22. Verfahren nach einem der Ansprüche 17 bis 21,
**dadurch gekennzeichnet,**
**dass** zur Messung der Topographie und/oder zur Messung der Wellenaberration ein Scanning-Spot-Verfahren herangezogen wird.

**Claims**

1. Device (1) for measuring the topography and wave aberration of a lens system (2),

   - having a first measuring system (8) that comprises a light source (10) for emitting a first light bundle (11) of a first wavelength ($\lambda$1), and a detector (20, 48) for picking up the first light bundle (11') reflected at the lens system (2), and
   - having a second measuring system (9) that comprises a light source (21) for emitting a second light bundle (22) of a second wavelength ($\lambda$2) and a detector (24, 48) for picking up the second light bundle (22') transmitted through the lens system (2),

   **characterized**
   **in that** there is arranged in a common beam path region (14) of the first measuring system (8) and of the second measuring system (9) a diffractive optical element (18) that adapts the respective wavefront profile (34) of the first light bundle (11, 11') and of the second light bundle (22, 22') in a wavelength-selective fashion.

2. Device (1) according to Claim 1,
   **characterized**
   **in that** the diffractive optical element (18) is designed to the effect that the zeroth diffraction order (31) of the first light bundle (11, 11') is suppressed, while the second light bundle (22, 22') is transmitted substantially undisturbed into the zeroth diffraction order (31).

3. Device (1) according to Claim 1 or 2,
   **characterized**
   **in that** the diffractive optical element (18) is designed to the effect that the wavefront profile (34) of the first light bundle (11, 11') is preadapted to the topography of the lens system (2).

4. Device (1) according to one of Claims 1 to 3,
   **characterized**
   **in that** the wavefront profile (34) of the second light bundle (22, 22') is substantially unmodified by the diffractive optical element (18).

5. Device (1) according to one of Claims 1 to 4,
   **characterized**
   **in that** the diffractive optical element (18) is designed as a surface-corrugated phase element.

6. Device (1),according to one of Claims 1 to 5,
   **characterized by**
   a third light source (38) for emitting a third light bundle (39) of a third wavelength ($\lambda$3) that is designed for inserting a fixation target into the lens system (2).

7. Device (1) according to Claim 6,
   **characterized**
   **in that** the third wavelength ($\lambda$3) corresponds to the second wavelength ($\lambda$2).

8. Device (1) according to one of Claims 1 to 7,
   **characterized**
   **in that** a common detector (48) is provided for the first measuring system (8) and the second measuring system (9).

9. Device (1) according to one of Claims 1 to 8,
   **characterized**
   **in that** at least the first wavelength ($\lambda$1) lies in the infrared spectral region.

10. Device (1) according to one of Claims 1 to 9,
    **characterized by**
    an adjusting arrangement (42) for setting the position of the lens system (2) with reference to the diffractive optical element (18).

**11.** Device (1) according to Claim 10,
**characterized**
**in that** the adjusting arrangement (42) comprises a light source (43) by means of which an adjusting light beam (45) can be cast onto the lens system (2) at an angle, and a position-sensitive detector (44) for determining the position of the adjusting light beam (45) reflected at the lens system (2).

**12.** Device (1) according to one of Claims 1 to 11,
**characterized**
**in that** the diffractive optical element (18) is directly upstream of the lens system (2).

**13.** Device (1) according to one of Claims 1 to 12,
**characterized**
**in that** the common beam path region (14) is limited by a wavelength-selective beam splitter (13).

**14.** Device (1) according to Claim 13,
**characterized**
**in that** the beam splitter (13) is designed as a diffractive optical element.

**15.** Device (1) according to one of Claims 1 to 14,
**characterized**
**in that** the detector (20, 48) of the first measuring system (8) and/or the detector (24, 48) of the second measuring system (9) is a Shack-Hartmann sensor.

**16.** Device (1) according to one of Claims 1 to 15,
**characterized**
**in that** the detector (20, 48) of the first measuring system (8) and/or the detector (24, 48) of the second measuring system (9) is an interferometer.

**17.** Method for measuring the topography and wave aberration of a lens system (2) in which a first light bundle (11) of a first wavelength ($\lambda$1) is cast onto the lens system (2) for the purpose of measuring the topography, and the first light bundle (11') reflected at the lens system (2) is detected, and in which a second light bundle (22) of a second wavelength ($\lambda$2) is cast onto the lens system (2) for the purpose of measuring the wave aberration, and the second light bundle (22') transmitted through the lens system (2) is detected,
**characterized**
**in that** the respective wavefront profile (34) of the first light bundle (11, 11') and of the second light bundle (22, 22') is adapted in a wavelength-selective fashion by a diffractive optical element (18) positioned in a common beam path region (14) of the first light bundle (11, 11') and of the second light bundle (22, 22').

**18.** Method according to Claim 17,
**characterized**
**in that** the first wavelength ($\lambda$1) and the second wavelength ($\lambda$2) are selected with regard to the diffractive optical element (18) in such a way that the zeroth diffraction order (31) of the first light bundle (11, 11') is suppressed by the diffractive optical element (18), while the second light bundle (22, 22') is transmitted substantially unattenuated into the zeroth diffraction order (31).

**19.** Method according to Claim 17 or 18,
**characterized**
**in that** the wavefront profile (34) of the first light bundle (11) is preadapted to the topography of the lens system (2) by the diffractive optical element (18).

**20.** Method according to one of Claims 17 to 19,
**characterized**
**in that** the topography and the wave aberration are measured simultaneously.

**21.** Method according to one of Claims 17 to 20,
**characterized**
**in that** the topography and the wave aberration are measured sequentially in time.

**22.** Method according to one of Claims 17 to 21,
**characterized**
**in that** a scanning spot method is used in order to measure the topography and/or the wave aberration.


**Revendications**

**1.** Dispositif (1) pour mesurer la topographie et l'aberration d'onde d'un système de lentille (2),

- comportant un premier système de mesure (8), qui comprend une source de lumière (10) pour irradier un premier faisceau de lumière (11) d'une première longueur d'onde ($\lambda$1) ainsi qu'un détecteur (20, 48) pour recevoir le premier faisceau lumineux (11') réfléchi sur le système de lentille (2), et
- comportant un second système de mesure (9), qui comprend une source de lumière (21) pour irradier un second faisceau lumineux (22) d'une seconde longueur d'onde ($\lambda$2) ainsi qu'un détecteur (24, 48) pour recevoir le second faisceau lumineux (22') transmis par le système de lentille (2),

**caractérisé en ce que**
un élément optique de diffraction (18) est disposé dans une zone de trajectoires des rayons commune (14) du premier système de mesure (8) et du second système de mesure (9), qui adapte l'allure du front d'onde respectif (34) du premier faisceau lumineux (11, 11') et du second faisceau lumineux (22, 22') de façon sélective par rapport à la longueur d'onde.

**2.** Dispositif (1) selon la revendication 1, **caractérisé en ce que**
l'élément optique de diffraction (18) est réalisé en ce que l'ordre de diffraction 0 (31) du premier faisceau lumineux (11, 11') est supprimé, tandis que le second faisceau lumineux (22, 22') est transmis sensiblement inchangé dans l'ordre de diffraction 0 (31).

**3.** Dispositif (1) selon la revendication 1 ou 2, **caractérisé en ce que**
l'élément optique de diffraction (18) est réalisé **en ce que** l'allure du front d'onde (34) du premier faisceau lumineux (11, 11') est pré-adapté à la topographie du système de lentille (2).

**4.** Dispositif (1) selon l'une des revendications 1 à 3, **caractérisé en ce que**
l'allure du front d'onde (34) du second faisceau lumineux (22, 22') n'est pas modifié sensiblement par l'élément optique de diffraction (18).

**5.** Dispositif (1) selon l'une des revendications 1 à 4, **caractérisé en ce que**
l'élément optique de diffraction (18) est réalisé sous la forme d'un élément de phase à surface ondulée.

**6.** Dispositif (1) selon l'une des revendications 1 à 5, **caractérisé par**
une troisième source de lumière (38) pour irradier un troisième faisceau lumineux (39) d'une troisième longueur d'onde ($\lambda$3), qui est réalisé pour l'incrustation d'une cible de fixation dans le système de lentille (2).

**7.** Dispositif (1) selon la revendication 6, **caractérisé en ce que**
la troisième longueur d'onde ($\lambda$3) correspond à la seconde longueur d'onde ($\lambda$2).

**8.** Dispositif (1) selon l'une des revendications 1 à 7, **caractérisé en ce que**
un détecteur commun (48) est prévu pour le premier système de mesure (8) et le second système de mesure (9).

**9.** Dispositif (1) selon l'une des revendications 1 à 8, **caractérisé en ce que**
au moins la première longueur d'onde ($\lambda$1) se trouve dans le domaine spectral infrarouge.

**10.** Dispositif (1) selon l'une des revendications 1 à 9, **caractérisé par**
un dispositif d'alignement (42) pour le réglage de la position du système de lentille (2) par rapport à l'élément optique de diffraction (18).

**11.** Dispositif (1) selon la revendication 10, **caractérisé en ce que**
le dispositif d'ajustement (42) comprend une source de lumière (43), grâce à laquelle un rayon lumineux d'ajustement (45) peut être projeté sur le système de lentille (2) selon un angle, ainsi qu'un détecteur sensible à la position (44) pour déterminer la position du rayon lumineux d'ajustement (45) réfléchi sur le système de lentille (2).

**12.** Dispositif (1) selon l'une des revendications 1 à 11, **caractérisé en ce que**
l'élément optique de diffraction (18) est monté immédiatement avant le système de lentille (2).

**13.** Dispositif (1) selon l'une des revendications 1 à 12, **caractérisé en ce que**
la zone de trajectoires des rayons commune (14) est limitée par un diviseur de rayons (13) de façon sélective par rapport à la longueur d'onde.

**14.** Dispositif (1) selon la revendication 13, **caractérisé en ce que**
le diviseur de rayons (13) est réalisé sous la forme d'un élément optique de diffraction.

**15.** Dispositif (1) selon l'une des revendications 1 à 14, **caractérisé en ce que**
le détecteur (20, 48) du premier système de mesure (8) et/ou le détecteur (24, 48) du second système de mesure (9) est un capteur de Shack-Hartmann.

**16.** Dispositif (1) selon l'une des revendications 1 à 15, **caractérisé en ce que**
le détecteur (20, 48) du premier système de mesure (8) et/ou le détecteur (24, 48) du second système de mesure (9) est un interféromètre.

**17.** Procédé pour mesurer la topographie et l'aberration d'onde d'un système de lentille (2) dans lequel un premier faisceau lumineux (11) d'une première longueur d'onde ($\lambda 1$) est projeté sur le système de lentille (2) pour mesurer la topographie, et le premier faisceau lumineux (11') réfléchi sur le système de lentille (2) est détecté, dans lequel un second faisceau lumineux (22) d'une seconde longueur d'onde ($\lambda 2$) est projeté sur le système de lentille (2) pour mesurer l'aberration d'onde, et le second faisceau lumineux (22') transmis par le système de lentille (2) est détecté, **caractérisé en ce que**
l'allure du front d'onde respective (34) du premier faisceau lumineux (11, 11') et du second faisceau lumineux (22, 22') est adaptée de façon sélective par rapport à la longueur d'ondes par un élément optique de diffraction (18) positionné dans une zone de trajectoires des rayons commune (14) du premier faisceau lumineux (11, 11') et du second faisceau lumineux (22, 22').

**18.** Procédé selon la revendication 17, **caractérisé en ce que**
la première longueur d'onde ($\lambda 1$) et la seconde longueur d'onde ($\lambda 2$) concernant l'élément optique de diffraction (18) sont choisies de sorte que l'ordre de diffraction 0 (31) du premier faisceau lumineux (11, 11') est supprimé par l'élément optique de diffraction (18), tandis que le second faisceau lumineux (22, 22') est transmis de façon sensiblement non affaiblie dans l'ordre de diffraction 0 (31).

**19.** Procédé selon la revendication 17 ou 18, **caractérisé en ce que**
l'allure du front d'onde (34) du premier faisceau lumineux (11) est pré-adaptée à la topographie du système de lentille (2) par l'élément optique de diffraction (18).

**20.** Procédé selon l'une des revendications 17 à 19, **caractérisé en ce que**
la mesure de la topographie et la mesure de l'aberration d'onde sont effectuées simultanément.

**21.** Procédé selon l'une des revendications 17 à 20, **caractérisé en ce que**
la mesure de la topographie et la mesure de l'aberration d'onde sont effectuées temporellement séquentiellement.

**22.** Procédé selon l'une des revendications 17 à 21, **caractérisé en ce que**
un procédé par balayage par spots est utilisé pour la mesure de la topographie et/ou la mesure de l'aberration d'onde.

Fig. 1

Fig. 2

Fig. 3

Fig. 4

Fig. 5

Fig. 6

Fig. 7

Fig. 8

**EP 1 662 981 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20020163623 A1
- US 20010016695 A1

- US 20030038921 A1